# EUROPEAN PATENT APPLICATION

(11) **EP 0 958 827 A1**
(43) Date of publication of application: **24.11.1999**
(21) Application number: 97903391.7
(22) Date of filing: 06.02.1997
(51) Int. Cl.: A61K 35/78

(54) **PROCESS FOR THE PREPARATION OF A NATURAL MEDICINAL LIQUOR BASED ON CARICA PAPAYA AND VINCA ROSEA**

(71) Applicant: Sesma Aizpurua, Manuel, 20750 Zumaya (ES)
(72) Inventor: Sesma Aizpurua, Manuel, 20750 Zumaya (ES)
(74) Representative: Perez Bonal, Bernardo
(86) International application number: ES9700027
(87) International publication number: WO9834627

(57) **Abstract**

The process comprises using a bacteriostatic sugar cane spirits, the latex of a fruit obtained by incision and further collected and which is macerated appropriately in said spirits, dissolving it with a mixer until a homogenous product is obtained; then the product is filtrated, and the same operation is carried out with flowers, both being immersed in a magnetic field during the maceration to obtain, after the final filtration, a non toxic liquor having a anti-tumoral activity.

## Description

### OBJECT OF THE INVENTION

The invention currently proposed consists of a PROCEDURE FOR OBTAINING A NATURAL MEDICINAL LIQUOR AND LIQUOR SO OBTAINED, from the liquors obtained from a maceration process of botanical products

The preparation in its composition, uses:
- Rum as a bacteriatic preservative
- Latex of *carica papaya* fruit, which is obtained by an incision followed by collection and which is then appropriately macerated in the aforementioned rum, dissolving with a mixer until achieving homogeneity; after which filtration is carried out.
- Flowers of the vinca rosea, macerated on the mentioned rum; after which filtration is carried out.

The mixture of both these ingredients is immersed in a magnetic field during the maceration, for which a magnet of the following characteristics is used:
- Permanent magnet of parallelepipedic form, measuring 45 mm x 50 mm x 10 mm.
- Intensity of magnetic field between 600 and 1100 Gauss.

As a final result a product is obtained which on the basis of the assays carried out *in vitro,* is considered to be non-toxic and to have anti-tumour activity.

### BACKGROUND OF INVENTION

The use of natural products for treatment of diseases of the immune system has been known for a long time and such products are used with different degrees of success in a great number of diseases.

A distillation of a certain type of rose is used in central European clinics, as well as a variety of mistletoe, in particular *viscum album,* cut according to a set ritual at a certain time. It is subjected to a complex extraction process, in which the extract, a castor-oil-like toxin, which inhibits protein synthesis by eukariotic cellsis distilled by the Hiscia Institute, Arlesheim (CH).

The product does not come into contact with products that are not natural at any time, and has been widely used in Switzerland and Germany for seven decades now. New experiments have been carried out during this decade at the University of Southern California (USA) with the *viscum coloratum,* in patients with AIDS.

More recently, also in Central Europe, mucopolysaccharides (beta 1,4 -manane) from a cactus of the aloe family is used, for example the vera of the Canary Islands. Others have also been used, such as mitraeformis, some of which have been widely used since the time of the Greeks and Romans and which are currently used for treating cancers, leukaemias, diseases related to old age, as well as inflammatory diseases of every type.

With regard to the components of the product object of the present invention, it can be said that studies on the characteristics of every one of them have been carried out. It is also known that the *carica papaya* is a vegetal pepsin with a high enzymatic value applied to tumours. The *vinca rosea* has applications as an anti-leukaemia agent, and rum as a dilution agent used as the best bacteriostatic preservative for maceration processes.

We should add to the above that the active components of the plants are metabolic products of a live organism and so a large number of them are assimilated by the human body in a very natural way and so are less damaging than products synthesised in the laboratory. For many years it has been known that a large number of the medicinal plants contain other valuable components, apart from the active ones. Some of these other components act in parallel with the active one, with a beneficial effect for the patient.

### DESCRIPTION OF INVENTION

The invention object of the present invention relates to a procedure for the preparation of a natural medicinal liquor, with a caramel colour, obtained by maceration followed by mixing of botanical products.

This invention is characterised by obtaining a preparation, on the basis of a macerate in rum of fruit latex, of *carica papaya* and of flowers of the *vinca rosea.* Then these components are mixed.

The latex of the fruit is obtained by making surface incisions in the fruit without removing it from the plant. These incisions are made with a scalpel using a beaker for collection. Beforehand, healthy fruits that have not been attacked by insects for other diseases will have been selected, and the fruit and collection beaker will have been disinfected.

The flowers are collected from uncontaminated land, using sterilised nylon envelopes.

The liquid obtained from this mixture is maintained immersed in a magnetic field for at least two days, after which the filtration is carried out, so obtaining a non-toxic product, with no secondary effects, with anti-tumour activity. It is beneficial for ulcers and diseases of the immune system of humans.

As a result of the assays carried out on lung tumours, such as Sarcoma 37 and Ehrlich ascitic tumour in animals, more exactly in rats of NMRI lineage, a reduction of 65% of the metastasis is obtained with respect to the control group.

In other trials, carried out for example on Lewis tumours in highly metastising strains, the amount of metastasis of the group treated was reduced from 32.8 to 28 % after 8 days of treatment.

Carrying out other assays confirms the anti-tumour activity of the product, preferentially on lung, prostate or bone tumours as well as on leukaemias.

Experience gained from trials carried out over the years suggests that cases of gastroduodenal ulcers and diabetic cases can benefit considerably from treatment.

On the other hand the product has been tested in isolated cases of AIDS and, taking into account complementary examinations, the results obtained are fairly promising with regard to the increase in haemoglobin and platelets, which could benefit the immunological system of the human organism.

### DESCRIPTION OF THE FIGURES

To complete the description that is being made and with the aim of improving and facilitating the comprehension of the characteristics of the invention, the present descriptive memorandum is accompanied as an integral part of it by a diagram in which, by way of illustration and never limiting, the following has been represented:

The figure of the schematic diagram shows in graphic form the steps of the procedure of the invention, up to when the final product is obtained.

### PREFERRED EMBODIMENT OF THE INVENTION

This invention relates to a procedure for the preparation of a natural medicinal liquor, obtained by means of maceration of latex of the *carica papaya* fruit and the flowers of the *vinca rosea,* from which a botanical product is obtained which is characterised as a preparation based on rum of between 35° and 40°; for this example in a volume of 1000 millilitres.

To do this the latex (1) of the selected fruits (I) is collected (IV) in a glass amber coloured recipient with a ground glass top. The selected fruit does not show any signs of disease, nor do the stalk or leaves. There is no evidence of insect attack or contamination. The latex is collected without removing the fruit from their branches, by means of washing (II) the fruit beforehand followed by the surface incisions in them (III) using a scalpel, until a quantity of between 50 and 120 grams has been collected. This quantity is mixed (V) with 650 millilitres of rum (2) using a mixer, until homogeneity is obtained. The mixture is macerated (VI) for at least two days, until the supernatant liquid (3) is translucent.

On the other hand the flowers (VII) are collected (VIII). These are cultivated on dry ground away from water sources, rivers or springs that might be contaminated that are protected from domestic animals. Complete flowers are harvested, with no signs of insect attack or contamination. These flowers are collected after blooming, during the early morning (9 am) and are placed (IX) in clean nylon envelopes, which are then closed and labelled (X) with their place of collection. Once between 150 and 280 grams have been selected, they are mixed (XI) with 350 millilitres of rum (2) using a mixer and macerated (XII) for at least two days until the flower completely loses its colour.

After these latex and flower maceration processes in rum, and after filtration (XIII) and (XIII'), these components are mixed (XIV) in an amber coloured glass recipient with a ground glass top, using a mixer until a characteristic caramel colour is attained.

Finally, a magnet (4) is introduced into this recipient, in the case of the example, with a parallelapipedic shape, 45 mm x 50 mm x 10 mm. The strength of the magnetic field varies between 600 and 1100 Gauss.

The resulting mixture is left to stand (XV) for at least two days while in contact with the magnet, which acts as a catalyst of the chemical reactions that occur in the mixture. This magnet is removed just before the final filtration is to be carried out (XVI), before the product (5) is bottled. The product is non-toxic, slightly sour to taste, with a cytostatic pH of 5. It is suitable for anti-tumour applications, with preference for lung tumours, prostate tumours, bone tumours, as well as leukaemias. The product is suitable and very beneficial in the treatment of gastroduodenal ulcers and diabetes. It preferentially acts on the immunological system in general.

Alternatively, the product can be obtained from a macerate in rum of latex of fruit and flowers. For this a recipient is used in which the rum is placed. Then the latex of fruit and the flowers are introduced, and everything mixed with a mixer. A magnet is introduced into this mixture which is then left to stand for at least two days, while it macerates appropriately. Finally the residues are filtered. There characteristics of the materials used as well as the proportions are the same as the example.

This description is not made more extensive, on the understanding that any person expert in this material would have sufficient information to understand the extent of the invention and the advantages derived from it, as would be able to reproduce it.

Both the variation of materials and their form, size and disposition are liable to variation within what has been characterised.

The terms used during the description and their sense should always be considered in the non-limiting sense.

## Claims

1. Procedure for obtaining a natural medicinal liquor, from among the liquors obtained by means of maceration, which comprises:
- the collection (IV), in a glass amber coloured recipient with ground glass top, of latex (1) of the fruits selected (I) from the *carica papaya,* which do not show any signs of disease, nor in the stalk nor in the leaves, nor do they show signs of attack by insects or contamination, obtaining the latex without removing the fruit from the branches, by means of washing (II) the fruit previously and then making a surface incision (III) with a scalpel, until a suitable quantity has been obtained, of between 50 and 120 grams, which is mixed (V) with 650 millilitres of rum (2) of between 35° and 40°, using a mixer, until homogeneity is obtained whereupon the mixture is macerated (VI) for at least two days, until the supernatant liquid is translucent;
- the collection (VIII) of flowers (VII) of the *vinca rosea* cultivated on dry ground away from water sources, rivers or spring that are contaminated, and protected from the presence of domestic animals, with complete flowers, with no trace of insect attack or contamination; these flowers are collected after blooming, during the early morning (9 am) and placed (IX) in clean nylon envelopes, which are then closed and labelled (X) showing the place where they were collected; once between 150 and 280 grams has been selected they are mixed with 350 millilitres of rum (2) of between 35° and 40°, using a mixing and the mixture is macerated (XII) for at least two days until the flowers completely lose their colour;
- the filtration (XIII) and (XIII') of these macerates from the latex of the fruit and the flowers, in rum;
- the mixing (XIV) of both filtered products, in a glass recipient, using a mixer until a characteristic caramel colour is obtained;
- the introduction of a magnet (4) into this recipient;
- leaving the resulting mixture (XV) to stand for at least two days in direct contact with the magnet; and
- the final filtration (XVI) of the mixture, before final bottling (5).

2. Procedure for obtaining a natural medicinal liquor, according to the previous claim, characterised in that the magnet, is parallelepidedic and has a magnetic field strength varying between 600 and 1100 Gauss, such that it acts as a catalyst of chemical reactions that take place in the mixture, and which is withdrawn before the final filtration, giving as a result a non-toxic product, slightly sour to the taste, with a cytostatic pH of 5.

3. A procedure for obtaining a natural medicinal liquor, according to the previous claim, characterised in that, as an alternative to the previous order, the product is obtained based on a macerate mixture of latex of fruit and of the flowers, using a recipient in which the rum is deposited; next the latex of the fruit and the flowers are added, mixing with a mixer, and just one magnet is introduced into this mixture leaving it to stand for at least two days, macerating it appropriately and finally filtering the residues.
